(19) Europäisches Patentamt
European Patent Office
Office européen des brevets

(11) **EP 1 188 839 A1**

(12) **EUROPEAN PATENT APPLICATION**

(43) Date of publication:
20.03.2002 Bulletin 2002/12

(51) Int Cl.7: **C12Q 1/68**, A61K 31/70,
A61K 45/00, A01K 67/027,
G01N 33/68

(21) Application number: 00120448.6

(22) Date of filing: 19.09.2000

(84) Designated Contracting States:
**AT BE CH CY DE DK ES FI FR GB GR IE IT LI LU
MC NL PT SE**
Designated Extension States:
**AL LT LV MK RO SI**

(71) Applicant: **EVOTEC Neurosciences GmbH
22525 Hamburg (DE)**

(72) Inventor: **The designation of the inventor has not
yet been filed**

(74) Representative:
**Meyers, Hans-Wilhelm, Dr.Dipl.-Chem. et al
Patentanwälte
von Kreisler-Selting-Werner
Postfach 10 22 41
50462 Köln (DE)**

Remarks:
The sequence listing, which is published as annex
to the application documents, was filed after the date
of filing. The applicant has declared that it does not
include matter which goes beyond the content of the
application as filed.

(54) **Diagnostic and therapeutic use of a caveolae-associated integral membrane protein for alzheimer's disease and related neurodegenerative disorders**

(57) The present invention discloses the differential expression of the caveolae-associated integral membrane protein flotillin-1 gene in specific brain regions of Alzheimer's disease patients. Based on this finding, this invention provides a method for diagnosing or prognosing Alzheimer's disease or other neurodegenerative diseases in a patient, or for determining whether a subject is at increased risk of developing Alzheimer's disease or other related neurodegenerative diseases. Furthermore, this invention provides therapeutic and prophylatic methods for treating or preventing Alzheimer's disease and related neurodegenerative disorders using a caveolae-associated integral membrane protein gene, in particular the flotillin-1 gene. A method of screening for modulating agents of neurodegenerative diseases is also disclosed.

## Figure 3: Verification of Differential Expression of Flotillin-1 by Quantitative RT-PCR

**Description**

The present invention relates to methods of diagnosing, prognosing and monitoring the progression of neurodegenerative diseases in a subject. Furthermore, methods of therapy control and screening for modulating agents of neurodegenerative diseases are provided. The invention also discloses pharmaceutical compositions, kits and recombinant animal models.

Neurodegenerative diseases, in particular Alzheimer's disease, have a strongly debilitating impact on a patient's life. Furthermore, these diseases constitute an enormous health, social and economic burden. Alzheimer's disease is the most common age-related neurodegenerative condition affecting about 10 % of the population over 65 years of age and up to 45 % over age 85 (for a recent review see Vickers et al., *Progress in Neurobiology* 2000, 60: 139-165). Presently this amounts to an estimated 12 million cases in the US, Europe, and Japan. This situation will inevitably worsen with the demographic increase in the number of old people ("aging of the baby boomers") in developed countries. The neuropathological hallmarks that occur in the brains of individuals with Alzheimer's disease are senile plaques, composed of amyloid-β protein, and profound cytoskeletal changes coinciding with the appearance of abnormal filamentous structures and the formation of neurofibrillary tangles. AD is a progressive disease that is associated with early deficits in memory formation and ultimately leads to the complete erosion of higher cognitive function. A characteristic feature of the pathogenesis of AD is the selective vulnerability of particular brain regions and subpopulations of nerve cells to the degenerative process. Specifically, the temporal lobe region and the hippocampus are affected early and more severely during the progression of the disease. On the other hand, neurons within the frontal cortex, occipital cortex, and the cerebellum remain largely intact and are protected from neurodegeneration (Terry et al., *Annals of Neurology* 1981, 10:184-192).

Currently, there is no cure for AD, nor is there an effective treatment to halt the progression of AD or even to diagnose AD ante-mortem with high probability. Several risk factors have been identified that predispose an individual to develop AD, among them most prominently the epsilon4 allele of apolipoprotein E (ApoE). Although there are rare examples of early-onset AD which have been attributed to genetic defects in the genes for APP, presenilin-1, and presenilin-2, the prevalent form of late-onset sporadic AD is of hitherto unknown etiologic origin. The late onset and complex pathogenesis of neurodegenerative disorders pose a formidable challenge to the development of therapeutic and diagnostic agents. Therefore, it is crucial to expand the pool of potential drug targets and diagnostic markers.

It is therefore an object of the present invention to provide insight into the pathogenesis of neurological diseases and to provide methods, material and animal models which are suited inter alia for the diagnosis and development of a treatment of these diseases.

This object has been solved by the features of the independant claims. The subclaims define preferred embodiments of the present invention.

The term "and/or" used in the present specification and the claims implies that the phrases before and after this term are considered either as alternatives or as combination. For instance, the wording "determination of a level and/or an activity" means that either only a level, or only an activity, or both a level and an activity are determined.

In one aspect, the invention features a method of diagnosing or prognosing a neurodegenerative disease in a subject, or determining whether a patient is at increased risk of becoming diseased with said disease. The method comprises: determining a level, or an activity, or both said level and said activity of (i) a transcription product of a gene coding for a caveolae-associated integral membrane protein, and/or of (ii) a translation product of a gene coding for a caveolae-associated integral membrane protein, and/or of (iii) a fragment or derivative of said transcription or translation product in a sample from said subject and comparing said level, and/or said activity to a reference value representing a known disease or health status, thereby diagnosing or prognosing said neurodegenerative disease in said subject, or determining whether said subject is at increased risk of developing said neurodegenerative disease.

In a further aspect, the invention features a method of monitoring the progression of a neurodegenerative disease in a subject. A level, or an activity, or both said level and said activity, of (i) a transcription product of a gene coding for a caveolae-associated integral membrane protein, and/or of (ii) a translation product of a gene coding for a caveolae-associated integral membrane protein, and/or of (iii) a fragment or derivative of said transcription or translation product in a sample from said subject is determined. Said level and/or said activity is compared to a reference value representing a known disease or health status. Thereby the progression of said neurodegenerative disease in said subject is monitored.

In still a further aspect, the invention features a method of evaluating a treatment for a neurodegenerative disease, comprising determining a level, or an activity, or both said level and said activity of (i) a transcription product of a gene coding for a caveolae-associated integral membrane protein, and/or of (ii) a translation product of a gene coding for a caveolae-associated integral membrane protein, and/or of (iii) a fragment or derivative of said transcription or translation product in a sample obtained from a subject being treated for said disease. Said level, or said activity, or both said level and said activity are compared to a reference value representing a known disease or health status, thereby evaluating the treatment for said neurodegenerative disease.

In a preferred embodiment, said subjects suffer from Alzheimer's disease. Further examples of neurodegenerative diseases are Parkinson's Disease, Huntington's disease, amytrophic lateral sclerosis, Pick's disease, frontotemporal dementia, progressive nuclear palsy, and cortical basal degeneration.

It is particularly preferred that said caveolae-associated integral membrane protein is a member of the flotillin gene family, specifically flotillin-1. It is further particularly preferred that said fragment of said caveolae-associated integral membrane protein is a fragment of flotillin-1, specifically FCRD.

This invention discloses the novel finding of the flotillin-1 gene as being differentially up-regulated in the temporal lobe region of Alzheimer's disease patients. Consequently, the flotillin-1 gene and and its corresponding translation products should have a causative role in the regional selective neuronal degeneration and/or confer a protective function to the remaining surviving nerve cells.

The gene for flotillin-1 was first cloned by Bickel et al. (*Journal of Biological Chemistry* 1997, 272:13793-13802; the contents of which are incorporated herein by reference) in an attempt to identify genes for novel proteins that are enriched in the membranes of purified caveolae from murine lung tissue. The cDNA for murine flotillin-1 encodes a protein of 428 amino acids with a predicted molecular weight of 47 kDa. The human flotillin-1 gene sequence (Genbank Accession No. AF 089750) was deposited in the Genbank data base by A.J. Edgar (Imperial College, London, UK) by direct submission in 1998. The human flotillin-1 gene encodes a protein of 427 amino acids. The amino acid sequences of the human and murine flotillin-1 protein are highly conserved (>98%). The flotillin-1 gene displays significant homology to ESA (epidermal surface antigen, also named flotillin-2). Although flotillin-1 and ESA have different N-termini, they share a region of 47% identity on the amino acid level. Furthermore, there is a modest (~24%) homology to two hypothetical open reading frames from the cyanobacterium *Synechococcus.* Flotillin-1 is expressed in most tissues and, contrary to ESA, also in brain. Bickel et al. concluded from Western blot experiments that there is evidence for a ~27 kDa immunoreactive protein named FCRD (= flotillin cross-reacting determinant). The origin of FCRD is not known. It could either be a degradation product generated during sample preparation or a physiologically relevant proteolytic fragment of the complete flotillin-1 protein.

Flotillin-1 is tightly associated with and enriched in caveolae membranes (i.e. an integral membrane protein). Caveolae ("little caves") are localized invaginations or vesicular organelles with a diameter of 50-100 μm, representing a functional subcompartment of the plasma membrane (for recent reviews see Anderson, *Annual Review of Biochemistry* 1998, 67:199-225 and Shaul and Anderson, *American Journal of Physiology* 1998, 275:L843-51). Caveolae have a relatively high content of cholesterol, sphingomyelin, glycosphingolipids and lipid-anchored membrane proteins. A light bouyant density and relative resistance to solubilization by detergents such as Triton X-100 are biochemical features of caveolae. Caveolins are the principal protein components of caveolae. It has been suggested that the caveolins (caveolin-1, caveolin-2, caveolin-3; all 21-24 kDa integral membrane proteins) concentrate and organize lipids and proteins into microdomains by functioning as scaffolding units. Additionally, many signal transduction molecules such as G-protein-coupled receptors, receptor tyrosine kinases, Src-family tyrosine kinases and nitric oxide synthase have been localized to caveolae. A direct role of caveolin-1 in the regulation of GPCR-mediated signaling cascades has been shown by Carman et al. (*J Biol Chem* 1999, 274:8858-64) and Schreiber et al. (*J Biol Chem* 2000, 275:24115-23). This implies that, altogether, caveolae serve a critical function in compartmentalizing, modulating, and integrating signalling events at the cell surface. Furthermore, caveolae are implicated in the regulation of vesicular transport processes and in sorting, processing and the degradation of internalized molecules.

The 45 kDa flotillin-1 protein is a marker protein for the slightly larger caveolae-related domains (50 - 200 nm). Flotillins are caveolae-associated integral membrane proteins. The flotillin gene family does not exhibit any sequence homology to the caveolins. Caveolin-1 and caveolin-3 each form homo-oligomers consisting of 14 - 16 monomers, and when caveolins and flotillins are co-expressed within the same cell they form stable hetero-oligomeric complexes (Volonté et al., *J Biol Chem* 1999, 274:12702-9). Furthermore, the heterologous expression of murine flotillin-1 in insect cells is sufficient to generate caveolae-like vesicles in cell culture experiments.

It is particularly preferred that said sample is a brain tissue or other body cells. The sample can also be cerebrospinal fluid or other body fluid including saliva, urine, and serum plasma.

In further preferred embodiments, said reference value is that of a level, or an activity, or both said level and said activity of (i) a transcription product of a gene coding for a caveolae-associated integral membrane protein, and/or of (ii) a translation product of a gene coding for a caveolae-associated integral membrane protein, and/or of (iii) a fragment or derivative of said transcription or translation product in a sample from a subject not suffering from said neurodegenerative disease.

In preferred embodiments, an increase or decrease in flotillin-1 mRNA and/or flotillin-1 protein in a cell or tissue from said subject relative to a reference value representing a known health status indicates a diagnosis, or prognosis, or increased risk of becoming diseased with a neurodegenerative disease, particularly Alzheimer's disease.

In preferred embodiments, measurement of the level of transcription products of a gene coding for a caveolae-associated integral membrane protein is performed in body cells using Northern blots with probes specific for said gene. Quantitative PCR with primer combinations to amplify said gene-specific sequences from cDNA obtained by reverse transcription of RNA extracted from body cells of a subject can also be applied. These techniques are known to those of ordinary skill in the art (see Sambrook and Russell, *Molecular Cloning: A Laboratory Manual,* Cold Spring Harbor Laboratory Press, Cold Spring Harbor, New York, 2000).

Furthermore, the level of a translation product of said gene and/or fragment of said translation product and/ or level of activity of said translation product and/or fragment of said translation product can be detected using an immunoassay, an enzyme activity assay, and/or binding assay. These assays can measure the amount of binding between said protein molecule and an anti-protein antibody by the use of enzymatic, chromodynamic, radioactive, or luminescent labels which are attached to either the anti-protein antibody or a secondary antibody which binds the anti-protein antibody. In addition, other high affinity ligands may be used. Immunoassays which can be used include e.g. ELISAs, Western blots and other techniques known to those of ordinary skill in the art (see Harlow and Lane, *Antibodies: A Laboratory Manual,* Cold Spring Harbor Laboratory Press, Cold Spring Harbor, New York, 1999).

In a preferred embodiment, the level, or the activity, or both said level and said activity of (i) a transcription product of a gene coding for a caveolae-associated integral membrane protein, and/or of (ii) a translation product of a gene coding for a caveolae-associated integral membrane protein, and/or of (iii) a fragment or derivative of said transcription or translation product in a series of samples taken from said subject over a period of time is compared, in order to monitor the progression of said disease. In further preferred embodiments, said subject receives a treatment prior to one or more of said sample gatherings. In yet another preferred embodiment, said level and/or activity is determined before and after said treatment of said subject.

In another aspect, the invention features a method of treating or preventing a neurodegenerative disease, in particular Alzheimer's disease, in a subject comprising the administration to said subject in a therapeutically or prophylactically effective amount of an agent or agents which directly or indirectly affect a level, or an activity, or both said level and said activity, of (i) a gene coding for a caveolae-associated integral membrane protein, and/or (ii) a transcription product of a gene coding for a caveolae-associated integral membrane protein, and/or (iii) a translation product of said gene, and/or (iv) a fragment or derivative of (i) to (iii).

In preferred embodiments, the method comprises the application of per se known methods of gene therapy and/or antisense nucleic acid technology to administer said agent or agents. In general, gene therapy includes several approaches: molecular replacement of a mutated gene, addition of a new gene resulting in the synthesis of a therapeutic protein, and modulation of endogenous cellular gene expression by recombinant expression methods or by drugs. Gene-transfer techniques are described in detail (see e.g. Behr, *Acc Chem Res* 1993, 26:274-278 and Mulligan, *Science,* 1993, 260: 926-931; the contents of which are incorporated herein by reference) and include direct gene-transfer techniques such as mechanical microinjection of DNA into a cell as well as indirect techniques employing biological vectors (like recombinant viruses, especially retroviruses) or model liposomes, or techniques based on transfection with DNA coprecipitation with polycations, cell membrane pertubation by chemical (solvents, detergents, polymers, enzymes) or physical means (mechanic, osmotic, thermic, electric shocks). The postnatal gene transfer into the central nervous system has been described in detail (see e.g. Wolff, *Curr Opin Neurobiol* 1993, 3:743-748).

In particular, the invention features a method of treating or preventing a neurodegenerative disease by means of antisense nucleic acid therapy, i.e. the down-regulation of an inappropriately expressed or defective gene by the introduction of antisense nucleic acids or derivatives thereof into certain critical cells (see e.g. Gillespie, *DN&P* 1992, 5:389-395; Agrawal and Akhtar, *Trends Biotechnol 1995,* 13:197-199; Crooke, *Biotechnology* 1992, 10:882-6; the contents of which are incorporated herein by reference). Apart from hybridization strategies, the application of ribozymes, i.e. RNA molecules that act as enzymes, destroying RNA that carries the message of disease has also been described (see e.g. Barinaga, *Science* 1993, 262:1512-1514; the contents of which are incorporated herein by reference). In preferred embodiments, the subject to be treated is a human, and therapeutic antisense nucleic acids or derivatives thereof are directed against a human caveolae-associated integral membrane protein, particularly flotillin-1. It is preferred that cells of the central nervous system, preferably the brain, of a subject are treated in such a way. Cell penetration can be performed by known strategies such as coupling of antisense nucleic acids and derivatives thereof to carrier particles, or the above described techniques. Strategies for administering targeted therapeutic oligodeoxynucleotides are known to those of skill in the art (see e.g. Wickstrom, *Trends Biotechnol,* 1992, 10: 281-287; the contents of which are incorporated herein by reference). In some cases, delivery can be performed by mere topical application. Further approaches are directed to intracellular expression of antisense RNA. In this strategy, cells are transformed *ex vivo* with a recombinant gene that directs the synthesis of an RNA that is complementary to a region of target nucleic acid. Therapeutical use of intracellularly expressed antisense RNA is procedurally similar to gene therapy.

In further preferred embodiments, the method comprises grafting donor cells into the central nervous system, preferably the brain, of said subject, or donor cells preferably treated so as to minimize or reduce graft rejection, wherein said donor cells are genetically modified by insertion of at least one transgene encoding said agent or agents. Said transgene might be carried by a viral vector, in particular a retroviral vector. The transgene can be inserted into the donor cells by a nonviral physical transfection of DNA encoding a transgene, in particular by microinjection. Insertion of the transgene can also be performed by electroporation, chemically mediated transfection, in particular calcium phosphate transfection, liposomal mediated transfection, etc.

In preferred embodiments, said agent is a therapeutic protein which can be administered to said subject, preferably a human, by a process comprising introducing subject cells into said subject, said subject cells having been treated *in vitro* to insert a DNA segment encoding said therapeutic protein, said subject cells expressing *in vivo* in said subject a therapeutically effective amount of said therapeutic protein. Said DNA segment can be inserted into said cells *in vitro* by a viral vector, in particular a retroviral vector.

In preferred embodiments, the subject can be a human, an experimental animal, e.g. a mouse or a rat, a domestic animal, or a non-human primate, e.g. a monkey. The experimental animal can be an animal model for a neurodegenerative disorder, e.g. a transgenic mouse with an Alzheimer's-type neuropathology.

In a further aspect, the invention features a modulator of an activity, or a level, or both said activity and said level of at least one substance which is selected from the group consisting of (i) a gene coding for a caveolae-associated integral membrane protein, and/or (ii) a transcription product of a gene coding for a caveolae-associated integral membrane protein, and/or (iii) a translation product of a gene coding for a caveolae-associated integral membrane protein, and/or (iv) a fragment or derivative of (i) to (iii).

In an additional aspect, the invention features a pharmaceutical composition comprising said modulator and preferably a pharmaceutical carrier. Said carrier refers to a diluent, adjuvant, excipient, or vehicle with which the modulator is administered.

In a further aspect, the invention features a modulator of an activity, or a level, or both said activity and said level of at least one substance which is selected from the group consisting of (i) a gene coding for a caveolae-associated integral membrane protein, and/or (ii) a transcription product of a gene coding for a caveolae-associated integral membrane protein, and/or (iii) a translation product of a gene coding for a caveolae-associated integral membrane protein, and/or (iv) a fragment or derivative of (i) to (iii) for use in a pharmaceutical composition.

In another aspect, the invention provides for the use of a modulator of an activity, or a level, or both said activity and said level of at least one substance which is selected from the group consisting of (i) a gene coding for a caveolae-associated integral membrane protein, and/or (ii) a transcription product of a gene coding for a caveolae-associated integral membrane protein, and/or (iii) a translation product of a gene coding for a caveolae-associated integral membrane protein, and/or (iv) a fragment or derivative of (i) to (iii) for a preparation of a medicament for treating or preventing a neurodegenerative disease, in particular Alzheimer's disease.

In one aspect, the present invention also provides a kit comprising one or more containers filled with a therapeutically or prophylactically effective amount of said pharmaceutical composition.

In a further aspect, the invention features a recombinant, non-human animal comprising a non-native gene sequence coding for a caveolae-associated integral membrane protein, or a fragment thereof, or a derivative thereof. The generation of said recombinant, non-human animals comprises (i) providing a gene targeting construct containing said gene sequence and a selectable marker sequence, and (ii) introducing said targeting construct into a stem cell of a non-human animal, and (iii) introducing said non-human animal stem cell into a non-human embryo, and (iv) transplanting said embryo into a pseudopregnant non-human animal, and (v) allowing said embryo to develop to term, and (vi) identifying a genetically altered non-human animal whose genome comprises a modification of said gene sequence in both alleles, and (vii) breeding the genetically altered non-human animal of step (vi) to obtain a genetically altered non-human animal whose genome comprises a modification of said endogenous gene, wherein said gene is mis-expressed, or under-expressed, or over-expressed, and wherein said disruption or alteration results in said non-human animal exhibiting a predisposition to developing symptoms of neuropathology similar to a neurodegenerative disease, in particular Alzheimer's disease. Strategies and techniques for the generation and construction of such an animal are known to those of ordinary skill in the art (see e.g. Capecchi, *Science,* 1989, 244:1288-1292 and Hogan et al., 1994, *Manipulating the Mouse Embryo: A Laboratory Manual,* Cold Spring Harbor Laboratory Press, Cold Spring Harbor, New York; the contents of the foregoing are incorporated herein by reference).

In preferred embodiments, said recombinant, non-human animal comprises a non-native gene sequence coding for a member of the flotillin gene family, in particular the caveolae-associated integral membrane protein flotillin-1, or a fragment thereof, in particular FCRD.

In another aspect, the invention features an assay for screening for a modulator of neurodegenerative diseases, in particular Alzheimer's disease, or related diseases and disorders of one or more substances selected from the group consisting of (i) a gene coding for a caveolae-associated integral membrane protein, and/or (ii) a transcription product of a gene coding for a caveolae-associated integral membrane protein, and/or (iii) a translation product of a gene coding for a caveolae-associated integral membrane protein, and/or (iv) a fragment or derivative of (i) to (iii). This screening method comprises (a) contacting a cell with a test compound, and (b) measuring the activity, or the level, or both the activity and the level of one or more substances recited in (i) to (iv), and (c) measuring the activity, or the level, or both the activity and the level of said substances in a control cell not contacted with said test compound, and (d) comparing the levels of the substance in the cells of step (b) and (c), wherein an alteration in the activity and/or level of said substances in the contacted cells indicates that the test compound is a modulator of said diseases and disorders.

In one further aspect, the invention features a screening assay for a modulator of neurodegenerative diseases, in particular Alzheimer's disease, or related diseases and disorders of one or more substances selected from the group consisting of (i) a gene coding for a caveolae-associated integral membrane protein, and/or (ii) a transcription product of a gene coding for a caveolae-associated integral membrane protein, and/or (iii) a translation product of a gene coding for a caveolae-associated integral membrane protein, and/or (iv) a fragment or derivative of (i) to (iii), comprising (a) administering a test compound to a test animal which is predisposed to developing or has already developed a neurodegenerative disease or related diseases or disorders, and (b) measuring the activity and/or level of one or more substances recited in (i) to (iv), and (c) measuring the activity and/or level of said substances in a matched control animal which is equally predisposed to developing or has already developed said diseases and to which animal no such test compound has been administered, and (d) comparing the activity and/or level of the substance in the animals of step (b) and (c), wherein an alteration in the activity and/or level of substances in the test animal indicates that the test compound is a modulator of said diseases and disorders.

In a preferred embodiment, said test animal and/or said control animal is a recombinant, non-human animal which expresses a caveolae-associated integral membrane protein, or a fragment thereof, or a derivative thereof, under the control of a transcriptional regulatory element which is not the native caveloae-associated integral membrane protein gene transcriptional control regulatory element.

In another aspect, the present invention provides for an assay for screening a plurality of compounds for inhibition between a ligand and a caveolae-associated integral membrane protein, or a fragment or derivative thereof. Said screening assay comprises the steps of (i) adding a liquid suspension of said caveolae-associated integral membrane protein, or a fragment or derivative thereof, to a plurality of containers, and (ii) adding a plurality of compounds to be screened for said inhibition to said plurality of containers, and (iii) adding fluorescently labelled ligand to said containers, and (iv) incubating said caveolae-associated integral membrane protein, or said fragment or derivative thereof, and said compounds, and said fluorescently labelled ligand, and (v) measuring the amounts of fluorescence associated with said caveolae-associated integral membrane protein, or with said fragment or derivative thereof, and (vi) determining the degree of inhibition by one or more of said compounds of binding of said ligand to said caveolae-associated integral membrane protein, or said fragment or derivative thereof. Instead of utilizing a fluorescently labelled ligand, it might in some aspects be preferred to use any other optically detectable label known to the person skilled in the art, e.g. radioactive labels, and detect it accordingly.

In one final aspect, the invention features an assay for screening a plurality of compounds to determine the degree of binding of said compounds to a caveolae-associated integral membrane protein, or to a fragment or derivative thereof. Said screening assay comprises (i) adding a liquid suspension of said caveolae-associated integral membrane protein, or a fragment or derivative thereof, to a plurality of containers, and (ii) adding a plurality of fluorescently labelled compounds to be screened for said binding to said plurality of containers, and (iii) incubating said caveolae-associated integral membrane protein, or said fragment or derivative thereof, and said fluorescently labelled compounds, and (iv) measuring the amounts of fluorescence associated with said caveolae-associated integral membrane protein, or with said fragment or derivative thereof, and (v) determining the degree of binding by one or more of said compounds to said caveolae-associated integral membrane protein, or said fragment or derivative thereof. Also in this type of assay it might be preferred to use another type of label.

In all types of assays disclosed herein it is preferred to study a member of the flotillin gene family as said caveolae-associated integral membrane protein. It is particularly preferred to conduct screening assays with flotillin-1.

Other features and advantages of the invention will be apparent form the following description of figures and examples.

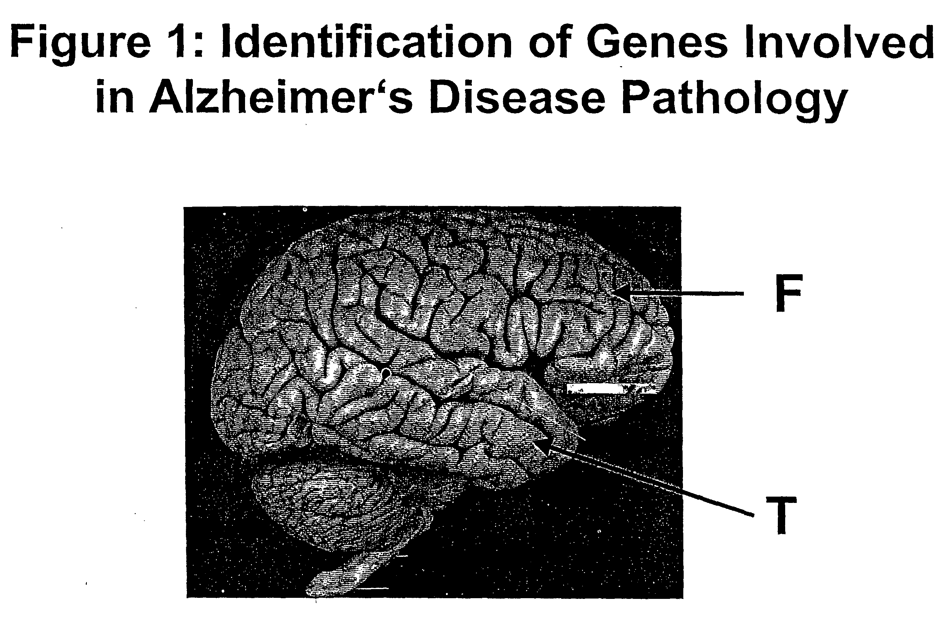

Figure 1 depicts the brain regions with selective vulnerability to neuronal loss and degeneration in Alzheimer's disease. Predominantly neurons within the inferior temporal lobe, the entorhinal cortex, the hippocampus, and the amygdala degenerate in Alzheimer's disease (Terry et al., *Annals of Neurology* 1981, 10:184-192). These brain regions are mostly involved in the processing of learning and memory functions. In contrast, neurons within the frontal cortex, the occipital cortex, and the cerebellum are largely intact and preserved from the neurodegenerative process in Alzheimer's disease. Brain tissue from the frontal cortex (F) and the temporal cortex (T) of Alzheimer's disease patients and healthy, age-matched control individuals was used for the herein disclosed examples. For illustrative purposes, the image of a normal, healthy brain was taken from a publication by Strange PG (1992, *Brain Biochem. Brain Disord.*).

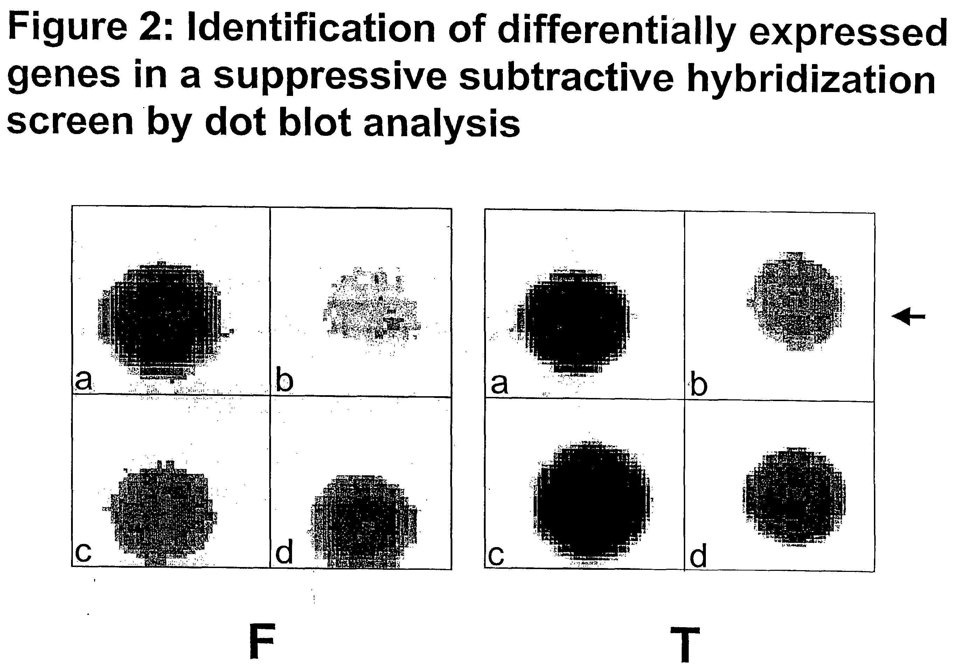

Figure 2 discloses the initial identification of the differential expression of flotillin-1 in a suppressive subtractive hybridization screen. The figure shows a clipping of a large-scale dot blot hybridization experiment. Individual cDNA clones from a temporally subtracted library were arrayed onto a nylon membrane and hybridized with probes enriched for genes expressed in the frontal cortex (F) and the temporal cortex (T) from an Alzheimer's disease patient. a) clone T10-Do6; b) flotillin-1; c) clone ens-0144; d) clone T10-E07. Note the significant increase in intensity of the hybridization signal for flotillin-1 in panel (T) (see arrow head) as compared to the signal in panel (F).

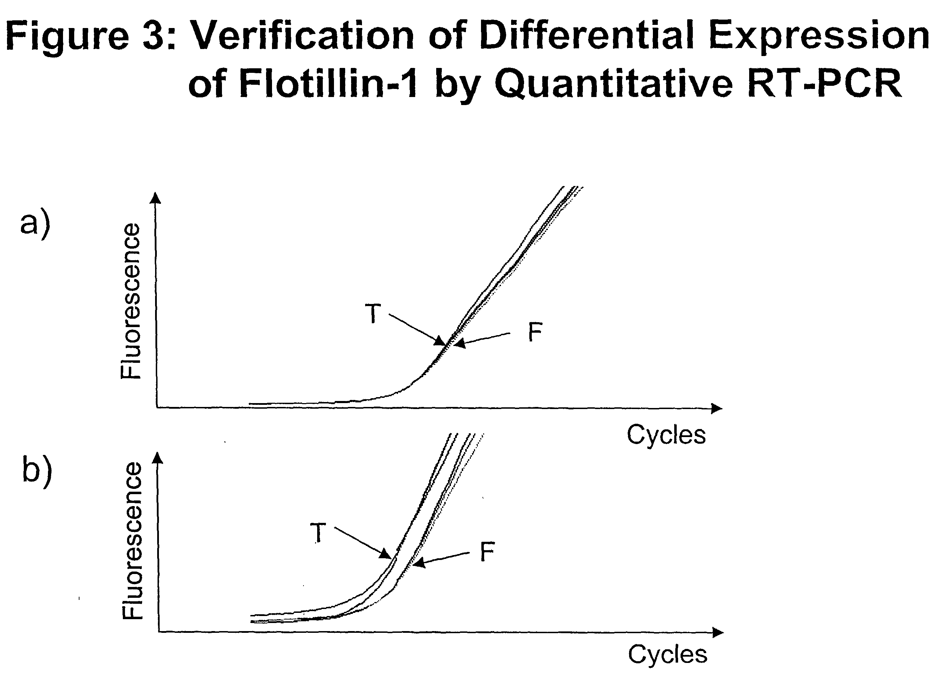

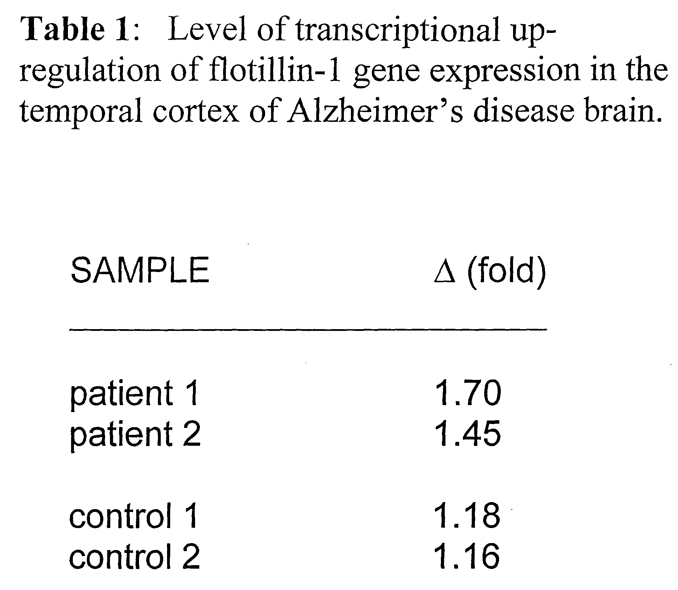

Figure 3 illustrates the verification of the differential expression of flotillin-1 by quantitative RT-PCR analysis. Quantification of RT-PCR products from RNA samples collected from the frontal cortex (F) and temporal cortex (T) of healthy, age-matched control individuals (Fig 3a) and Alzheimer's disease patients (Fig 3b) was performed by the LightCycler™ rapid thermal cycling technique. The data were normalized to cyclophilin B which showed no significant difference in its gene expression level. The figure depicts the kinetics of amplification by plotting the cycle number against the amount of amplified material as measured by its fluorescence. Note that the amplification kinetics of flotillin-1 cDNA from both the frontal and temporal cortices of a normal control individual during the exponential phase of the reaction overlap, whereas in Alzheimer's disease (Fig 3b) there is a significant shift of the curve for the sample from temporal cortex, indicating an up-regulation of flotillin-1 mRNA in this particular tissue in relation to the frontal cortex.

Table 1 lists the up-regulation of gene expression levels in the temporal cortex relative to the frontal cortex for the flotillin-1 gene in two Alzheimer's disease patients and two healthy, age-matched control individuals.

EXAMPLE I:

(i) Brain tissue dissection from patients with Alzheimer's disease:

Brain tissues from Alzheimer's disease patients and age-matched control subjects were collected within 6 hours of death and immediately frozen on dry ice. Sample sections from each tissue were fixed in paraformaldehyde for histopathological confirmation of the diagnosis. Brain areas for differential expression analysis were identified (see Fig. 1) and stored at -80 °C until RNA extractions were performed.

(ii) Isolation of total mRNA:

Total RNA was extracted from post-mortem brain tissue by using the RNeasy™ kit (Qiagen) according to the manufacturer's protocol. The quality of the prepared RNA was determined by formaldehyde agarose gel electrophoresis and Northern blotting according to standard procedures (Sambrook and Russell, *Molecular Cloning: A Laboratory Manual,* Cold Spring Harbor Laboratory Press, Cold Spring Harbor, New York, 2000). The mRNA was isolated from the total RNA preparation using the Quickprep Micro™ mRNA Purification Kit (Pharmacia Biotech) with yields between 1 and 5 %.

(iii) cDNA synthesis and identification of differentially expressed genes by suppressive subtractive hybridization:

This technique compares two populations of mRNA and provides clones of genes that are expressed in one population but not in the other. The applied technique was described in detail by Diatchenko et al. (*Proc Natl Acad Sci USA* 1996, 93:6025-30) In the present invention, mRNA populations from post-mortem brain tissues from Alzheimer's disease patients were compared. Specifically, mRNA of the frontal cortex was subtracted from mRNA of the inferior temporal cortex. The necessary reagents were taken from the PCR-Select™ cDNA subtraction kit (Clontech), and all steps were performed as described in the manufacturer's protocol. Specifically, 2µg mRNA each were used for first-strand and second-strand cDNA synthesis. After RsaI-digestion and adaptor ligation hybridization of tester and driver was performed for 8 hours (first hybridization) and 15 hours (second hybridization) at 68 °C. Two PCR steps were performed to amplify differentially expressed genes (first PCR: 27 cycles of 94 °C and 30 sec, 66 °C and 30 sec, and 72 °C and 1.5 min; nested PCR: 12 cycles of 94 °C and 30 sec, 66 °C and 30 sec, and 72 °C and 1.5 min) using adaptor specific primers (included in the subtraction kit) and 50x Advantage Polymerase Mix (Clontech). Efficiencies of RsaI-digestions, adaptor ligations and subtractive hybridizations were checked as recommended in the kit.

Subtracted cDNAs were inserted into the pCR® vector and transformed into *E.coli* INVαF' cells (Invitrogen).

To isolate individual cDNAs of the subtracted library single bacterial transformants were incubated in 100 µl LB (with 50 µg/ml ampicillin) at 37 °C for at least 4 hours. Inserts were PCR amplified (95 °C and 30 sec, 68 °C and 3 min for 30 cycles) in a volume of 20 µl containing 10 mM Tris-HCl pH 9.0, 1.5 mM MgCl$_2$, 50 mM KCl, 200 µM dNTP, 0.5 µM adaptor specific primers (included in the subtraction kit), 1.5 Units Taq polymerase (Pharmacia Biotech), and 1µl of bacterial culture. 1.5 µl of a mixture containing 3 µl PCR amplified inserts and 2 µl 0.3 N NaOH/15 % Ficoll were spotted onto a positively charged nylon membrane (Roche). In this way, hundreds of spots were arrayed on duplicate filters for subsequent hybridization. The differential screening step consisted of hybridizations of the subtracted library with itself to minimize background (Wang and Brown, *Proc Natl Acad Sci USA* 1991, 88:11505-9). The probes were made of the nested PCR product of the subtraction following the instructions of the Clontech subtraction kit. Labeling with digoxigenin was performed with the DIG DNA Labeling Kit (Roche). Hybridizations were carried out overnight in DIG Easy HYB (Roche) at 43 °C. The filters were washed twice in 2 x SSC / 0.5 % SDS at 68 °C for 15 min and twice in 0.1 x SSC / 0.5 % SDS at 68 °C for 15 min, and subjected to detection using anti-DIG-AP conjugates and CDP-Star™ as chemiluminescent substrate according to the instructions of the DIG DNA Detection Kit (Roche). Blots were exposed to Kodak Biomax MR chemiluminescent film at room temperature for several minutes. The results of this subtractive hybridization for the flotillin-1 gene are shown in Fig. 2.

(iv) Confirmation of differential expression by quantitative RT-PCR:

Positive confirmation of differential expression of the flotillin-1 gene was performed using the LightCycler™ technology (Roche). This technique features rapid thermal cyling for the polymerase chain reaction as well as real-time measurement of fluorescent signals during amplification and therefore allows for highly accurate quantification of RT-PCR products by using a kinetic, rather than an endpoint approach. The ratio of flotillin-1 cDNA from the temporal cortex and frontal cortex was determined (relative quantification).

First, a standard curve was generated to determine the efficiency of the PCR with specific primers for flotillin-1 (5'-CTGCCAGAGAGTGTGGAAAGACT-3' and 5'-TCTCAAAGGCTTGTGATTCACCT-3'). PCR amplification (95 °C and 1 sec, 56 °C and 5 sec, and 72 °C and 5 sec) was performed in a volume of 20 µl containing Lightcycler-DNA Master SYBR Green ready-to-use mix (contains Taq DNA polymerase, reaction buffer, dNTP mix with dUTP instead of dTTP, SYBR Green I dye, and 1 mM $MgCl_2$, Roche), additional 3 mM $MgCl_2$, 0,5 µM primers, 0,16 µl TaqStart® antibody (Clontech), and 1 µl of a cDNA dilution series (40, 20, 10, 5, and 1 ng human total brain cDNA, Clontech). Melting curve analysis revealed a single peak at approximately 83°C with no visible primer dimers. Agarose gel analysis of the PCR product showed one single band of the expected size (66 bp).

The same protocol was applied to determine the PCR efficiency of the reference gene, cyclophilin B, using the specific primers 5'-ACTGAAGCACTACGGGCCTG-3' and 5'-AGCCGTTGGTGTCTTTGCC-3' except for $MgCl_2$ (additional 1 mM was added instead of 3 mM). Cyclophilin-B was chosen for normalization because it was found to be the least regulated gene among all analyzed housekeeping genes. Melting curve analysis revealed a single peak at approximately 87 °C with no visible primer dimers. Agarose gel analysis of the PCR product showed one single band of the expected size (62 bp).

The logarithm of the cDNA concentration was plotted against the threshold cycle number $C_t$ for both flotillin-1 and cyclophilin B. The slopes and the intercepts of the standard curves (linear regressions) were calculated for both genes.

In a second step, cDNA from temporal cortex and frontal cortex was analyzed in parallel with cyclophilin B for normalization. The $C_t$ values were measured and converted to ng total brain cDNA using the corresponding standard curves:

$$10 \wedge ( (C_t \text{ value - intercept) / slope )} \qquad \text{[ng total brain cDNA]}$$

The values of temporal and frontal cortex flotillin-1 cDNAs were normalized to cyclophilin B and the ratio was calculated using the following formula:

$$\text{Ratio} = \frac{\text{flotillin-1 temporal [ng] / cyclophilin B temporal [ng]}}{\text{flotillin-1 frontal [ng] / cyclophilin B frontal [ng]}}$$

The results of a representative quantitative RT-PCR analysis for the flotillin-1 gene are shown in Fig. 3.

## Claims

1. A method of diagnosing or prognosing, monitoring the progression, evaluating a treatment for a neurodegenerative disease in a subject, or determining whether a subject is at increased risk of developing said disease, comprising:
   determining a level and/or an activity of

   (i) a transcription product of a gene coding for a caveolae-associated integral membrane protein, and/or

   (ii) a translation product of a gene coding for a caveolae-associated integral membrane protein, and/or

   (iii) a fragment or derivative of said transcription or translation product, in a sample from said subject and comparing said level and/or said activity to a reference value representing a known disease or health status, thereby

   diagnosing or prognosing said neurodegenerative disease in said subject,

   monitoring the progression of said neurodegenerative disease in said subject,

   evaluating said treatment for said neurodegenerative disease,

   or determining whether said subject is at increased risk of developing said neurodegenerative disease.

2. The method according to claim 1 wherein said neurodegenerative disease is Alzheimer's disease.

3. The method according to claim 1 or 2 wherein said caveolae-associated integral membrane protein is a member of the flotillin gene family, in particular flotillin- 1.

4. The method according to any of claims 1 to 3 wherein said fragment of said caveolae-associated integral membrane protein is a fragment of flotillin-1, in particular FCRD.

5. A modulator of an activity and/or of a level of at least one substance which is selected from the group consisting of (i) a gene coding for a caveolae-associated integral membrane protein, and/or (ii) a transcription product of a gene coding for a caveolae-associated integral membrane protein, and/or (iii) a translation product of a gene coding for a caveolae-associated integral membrane protein, and/or (iv) a fragment or derivative of (i) to (iii).

6. The pharmaceutical composition comprising a modulator according to claim 5.

7. A recombinant, non-human animal comprising a non-native gene sequence coding for a caveolae-associated integral membrane protein, or a fragment thereof, or a derivative thereof, said animal being obtainable by:

(i) providing a gene targeting construct comprising said gene sequence and a selectable marker sequence, and

(ii) introducing said targeting construct into a stem cell of a non-human animal, and

(iii) introducing said non-human animal stem cell into a non-human embryo, and

(iv) transplanting said embryo into a pseudopregnant non-human animal, and

(v) allowing said embryo to develop to term, and

(vi) identifying a genetically altered non-human animal whose genome comprises a modification of said gene sequence in both alleles, and

(vii) breeding the genetically altered mouse of step (vi) to obtain a genetically altered non-human animal whose genome comprises a modification of said endogenous gene, wherein said disruption results in said non-human animal exhibiting a predisposition to developing a neurodegenerative disease or related disease or disorders.

8. The animal according to claim 7 wherein said caveolae-associated integral membrane protein is a member of the flotillin gene family, in particular flotillin-1.

9. An assay for screening for a modulator of neurodegenerative diseases, in particular Alzheimer's disease, or related diseases or disorders of one or more substances selected from the group consisting of

(i) a gene coding for a caveolae-associated integral membrane protein, and/or

(ii) a transcription product of a gene coding for a caveolae-associated integral membrane protein, and/or

(iii) a translation product of a gene coding for a caveolae-associated integral membrane protein, and/or

(iv) a fragment or derivative of (i) to (iii), said method comprising:

(a) contacting a cell with a test compound;

(b) measuring the activity and/or level of one or more substances recited in

(c) measuring the activity and/or level of one or more substances recited in (i) to (iv) in a control cell not contacted with said test compound; and

(d) comparing the levels and/or activities of the substance in the cells of step (b) and (c), wherein an alteration in the activity and/or level of substances in the contacted cells indicates that the test compound is a modulator of said diseases or disorders.

10. A method of screening for a modulator of neurodegenerative diseases, in particular Alzheimer's disease, or related diseases or disorders of one or more substances selected from the group consisting of

(i) a gene coding for a caveolae-associated integral membrane protein, and/or

(ii) a transcription product of a gene coding for a caveolae-associated integral membrane protein, and/or

(iii) a translation product of a gene coding for a caveolae-associated integral membrane protein, and/or

(iv) a fragment or derivative of (i) to (iii), said method comprising:

(a) administering a test compound to a test animal which is predisposed to developing or has already developed a neurodegenerative disease or related diseases or disorders in respect of the substances recited in (i) to (iv);

(b) measuring the activity and/or level of one or more substances recited in (i) to (iv);

(c) measuring the activity and/or level of one or more substances recited in (i) or (iv) in a matched control animal which is predisposed to developing or has already developed a neurodegenerative disease or related diseases or disorders in respect to the substances recited in (i) to (iv) and to which animal no such test compound has been administered;

(d) comparing the activity and/or level of the substance in the animals of step (b) and (c), wherein an alteration in the activity and/or level of substances in the test animal indicates that the test compound is a modulator of said diseases or disorders.

11. The method according to claim 10 wherein said test animal and/or said control animal is a recombinant animal which expresses a caveolae-associated integral membrane protein, or a fragment thereof, or a derivative thereof, under the control of a transcriptional control element which is not the native caveolae-associated integral membrane protein gene transcriptional control element.

12. An assay for screening a plurality of compounds for inhibition between a ligand and a caveolae-associated integral membrane protein, or a fragment or derivative thereof, said assay comprising the steps of -

(i) adding a liquid suspension of said caveolae-associated integral membrane protein, or a fragment or derivative thereof, to a plurality of Containers

(ii) adding a plurality of compounds to be screened for said inhibition to said plurality of Containers

(iii) adding detectable ligand to said containers

(iv) incubating said caveolae-associated integral membrane protein, or said fragment or derivative thereof, and said compounds, and said detectable labelled ligand;

(v) measuring amounts of detectable ligand associated with said caveolae-associated integral membrane protein, or with said fragment or derivative thereof; and

(vi) determining the degree of inhibition by one or more of said compounds of binding of said ligand to said caveolae-associated integral membrane protein, or said fragment or derivative thereof.

13. An assay for screening a plurality of compounds to determine the degree of binding of said compounds to a caveolae-associated integral membrane protein, or to a fragment or derivative thereof, said assay comprising the steps of

(i) adding a liquid suspension of said caveolae-associated integral membrane protein, or a fragment or derivative thereof, to a plurality of Containers;

(ii) adding a plurality of detectable compounds to be screened for said binding to said plurality of containers;

(iii) incubating said caveolae-associated integral membrane protein, or said fragment or derivative thereof, and said detectable compounds;

(iv) measuring amounts of said detectable compounds associated with said caveolae-associated integral membrane protein, or with said fragment or derivative thereof, and

(v) determining the degree of binding by one or more of said compounds to said caveolae-associated integral membrane protein, or said fragment or derivative thereof.

Figure 1: Identification of Genes Involved in Alzheimer's Disease Pathology

# Figure 2: Identification of differentially expressed genes in a suppressive subtractive hybridization screen by dot blot analysis

F

T

# Figure 3: Verification of Differential Expression of Flotillin-1 by Quantitative RT-PCR

a)

b)

EP 1 188 839 A1

**Table 1**: Level of transcriptional up-regulation of flotillin-1 gene expression in the temporal cortex of Alzheimer's disease brain.

| SAMPLE | $\Delta$ (fold) |
|---|---|
| patient 1 | 1.70 |
| patient 2 | 1.45 |
| control 1 | 1.18 |
| control 2 | 1.16 |

# EUROPEAN SEARCH REPORT

**European Patent Office**

**Application Number**

EP 00 12 0448

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | NISHIYAMA K ET AL: "Caveolin-3 upregulation activates beta-secretase-mediated cleavage of the amyloid precursor protein in alzheimer's disease" THE JOURNAL OF NEUROSCIENCE, vol. 19, no. 15, August 1999 (1999-08), pages 6538-48, XP002171908 * the whole document * | 1,2 | C12Q1/68 A61K31/70 A61K45/00 A01K67/027 G01N33/68 |
| X | CHEMICAL ABSTRACTS, vol. 133, no. 21, 20 November 2000 (2000-11-20) Columbus, Ohio, US; abstract no. 294738y, KOBUBO K ET AL: "Localization of flotillins in human brain and their accumulation with the progression of alzheimer's disease pathology" page 551; column 1; XP002171911 * abstract * & KOKUBO H ET AL: NEUROSCIENCE LETTERS, vol. 290, no. 2, 2000, pages 93-6, | 1,2 | |
| A | VOLONTÉ D ET AL: "Flotillins/cavatellins are differentially expressed in cells and tissues and form hetero-oligomeric complex with caveolins in vivo" THE JOURNAL OF BIOLOGICAL CHEMISTRY, vol. 274, no. 18, 30 April 1999 (1999-04-30), pages 12702-9, XP002171909 * the whole document * | 1 | TECHNICAL FIELDS SEARCHED (Int.Cl.7) C12Q A01K A61K G01N |

-/--

~~The present search report has been drawn up for all claims~~

| Place of search | Date of completion of the search | Examiner |
|---|---|---|
| THE HAGUE | 13 July 2001 | OSBORNE, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent

Office

**Application Number**

EP 00 12 0448

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-4, 7-11

European Patent Office

**EUROPEAN SEARCH REPORT**

Application Number

EP 00 12 0448

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | BICKEL P ET AL: "Flotillin and epidermal surface antigen define a new family of caveolae-associated integral membrane proteins" THE JOURNAL OF BIOLOGICAL CHEMISTRY, vol. 272, no. 21, 23 May 1997 (1997-05-23), pages 13793-802, XP002171910 * the whole document * | 1 | |
| A | WO 97 18454 A (THOMPSON TIMOTHY) 22 May 1997 (1997-05-22) | | |

TECHNICAL FIELDS SEARCHED (Int.Cl.7)

The present search report has been drawn up for all claims

| Place of search | Date of completion of the search | Examiner |
|---|---|---|
| THE HAGUE | 13 July 2001 | OSBORNE, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent
Office**

**LACK OF UNITY OF INVENTION
SHEET B**

**Application Number**

EP 00 12 0448

The Search Division considers that the present European patent application does not comply with the requirements of unity of invention and relates to several inventions or groups of inventions, namely:

1. Claims: 1-4,7-11

    Method for diagnosing or prognosing, monitoring the progression and evaluating a treatment for a neurodegenerative disease by determining the level and/or activity of a transcription and/or translation product of a gene coding for a caveolae-associated integral membrane protein; an invitro cell-based method for screening for a modulator of a neurodegenerative disease by monitoring either of said gene product(s); a recombinant, non-human animal including a non native gene coding for said caveolae-associated integral membrane protein, and use thereof for screening a modulator of a neurodegenerative disease.

2. Claims: 5,6

    A modulator of an activity and /or level of a gene coding for an caveolae-associated integral membrane protein, and a pharmaceutical composition comprising a said modulator

3. Claims: 12,13

    An assay for screening for inhibitors between a ligand and a caveolae-associated integral membrane protein or a fragment thereof; an assay for determining the degree of binding of a compoung to said caveolae-associated integral membrane protein or a fragment thereof.

**ANNEX TO THE EUROPEAN SEARCH REPORT
ON EUROPEAN PATENT APPLICATION NO.**                    EP 00 12 0448

This annex lists the patent family members relating to the patent documents cited in the above-mentioned European search report.
The members are as contained in the European Patent Office EDP file on
The European Patent Office is in no way liable for these particulars which are merely given for the purpose of information.

13-07-2001

| Patent document cited in search report | | Publication date | Patent family member(s) | | Publication date |
|---|---|---|---|---|---|
| WO 9718454 | A | 22-05-1997 | US | 5783182 A | 21-07-1998 |
| | | | AU | 725186 B | 05-10-2000 |
| | | | AU | 1160997 A | 05-06-1997 |
| | | | CA | 2237929 A | 22-05-1997 |
| | | | EP | 0870057 A | 14-10-1998 |

EPO FORM P0459

For more details about this annex : see Official Journal of the European Patent Office, No. 12/82

Annex to the application documents - subsequently filed sequences listing

SEQUENCE LISTING

<110> Evotec Neurosciences GmbH

<120> Diagnostic and therapeutic use of a caveolae-associated
integral membrane protein for alzheimer´s disease and
related neurodegenerative disorders

<130> Evotec 00 120 448.6

<140>
<141>

<160> 4

<170> PatentIn Ver. 2.1

<210> 1
<211> 23
<212> DNA
<213> Artificial Sequence

<220>
<223> Description of Artificial Sequence: primer

<400> 1
ctgccagaga gtgtggaaag act                                              23

<210> 2
<211> 23
<212> DNA
<213> Artificial Sequence

<220>
<223> Description of Artificial Sequence: primer

<400> 2
tctcaaaggc ttgtgattca cct                                              23

<210> 3
<211> 20
<212> DNA
<213> Artificial Sequence

<220>
<223> Description of Artificial Sequence: primer

```
<400> 3
actgaagcac tacgggcctg                                           20


<210> 4
<211> 19
<212> DNA
<213> Artificial Sequence

<220>
<223> Description of Artificial Sequence: primer

<400> 4
agccgttggt gtctttgcc                                            19
```